# EUROPEAN PATENT APPLICATION

(11) **EP 3 875 159 A1**
(43) Date of publication of application: **08.09.2021**
(21) Application number: 19879811.8
(22) Date of filing: 30.10.2019
(51) Int. Cl.: A63B 69/00, A63B 69/16

(54) **MOTION-CAPTURE SYSTEM, MOTION-CAPTURE PROGRAM, AND MOTION-CAPTURE METHOD**

(30) Priority: 31.10.2018 JP 2018205106
(71) Applicant: Leomo, Inc., Boulder, Colorado 80301 (US)
(72) Inventor: KAJI Kunihiko, Tokyo 141-0031 (JP); FUKUMA Shinichi, Tokyo 141-0031 (JP)
(74) Representative: Grünecker Patent- und Rechtsanwälte PartG mbB
(86) International application number: PCT/JP2019/042598
(87) International publication number: WO 2020/090899

(57) **Abstract**

[Problem] To display/output a complex body motion by accurately synchronizing said body motion with motion image so as to allow a detailed interpretation through comparison thereof.

[Solution] This system is provided with: a plurality of body motion sensors 40 that are attached to arbitrarily defined sites of a wearer 1 so as to be capable of measuring three dimensional displacements and accelerations of the respective sites; an external camera 20 for photographing and recording a body motion of the wearer 1; a display unit that displays, as body motion data, detection results obtained by the body motion sensors 40 in synchronization with a video recorded by the external camera 20 in such a manner as to enable comparison therebetween; and a synchronization processing unit 170f that executes synchronization processing in which a time axis displaying the body motion data is coincided with a time axis displaying the video, wherein the synchronization processing unit 170f performs the synchronization processing by extracting a specific characteristic action performed by the wearer from within video or sound and also extracting a characteristic reaction in the body motion data and then matching the timing of the extracted characteristic action with the timing of the characteristic reaction.

## Description

### FIELD OF THE INVENTION

The present invention relates to a motion capture system, a motion capture program, and a motion capture method for detecting the body motion of a wearer by the use of a so-called smartphone, a wearable type device or another information terminal device.

### BACKGROUND ART

In recent years, along with the advent of the miniaturization, weight saving and multifunction of the information terminal device, wearing type information processing terminals, i.e., so-called wearable terminals are becoming spread, and such a wearable terminal is lightweight and provided with a clock function, a GPS function and other functions such as a communication function to perform communication with various sensors such as a heart rate sensor, so that systems have been developed which can be worn during running, walking, sports training or exercise such as riding a cycle for recording or monitoring body motion (for example, refer to Patent Document 1).

In accordance with the system disclosed in this Patent Document 1, it is possible to provide realtime feedback data to a user during exercise by attaching a device for measuring motion parameters to the user, comparing motion parameters obtained during monitoring the exercising user with basic baseline data, and changing playback music or the like in accordance with whether or not the result of comparison falls in an allowable range.

Incidentally, while motion can be recorded in detail as motion parameters which are represented by numerals and graphic expression, it is difficult to imagine the actual body motion therefrom, and therefore there is a need for confirming the body motion by capturing the body motion of an user who is exercising with a video camera or the like and comparing the captured motion image with the motion parameters.

### PRIOR ART DOCUMENTS

### Patent Document

Patent document 1:
Japanese Patent Published Application No. 2013-215590

### SUMMARY OF INVENTION

### Problems to be Solved by the Invention

However, since the body motion of a user instantaneously varies in a complicated manner during exercise, there is the possibility that a slight time lag may occur between the replayed motion image and the displayed motion parameters depending upon the characteristics of the photographing equipment and the motion image process. In the case where a lag occurs between the replayed motion image and the displayed motion parameters, there is a fear that appropriate training and coaching cannot be performed because of the degraded analysis accuracy of the body motion.

In order to solve the problem as described above, it is an object of the present invention to provide a motion capture system, a motion capture program and a motion capture method in which, when capturing exercise of the body of a user in the system, complicated body motions are accurately synchronized with a video image and displayed/output in order that the body motions can be understood in detail while comparing with the video image. Means to Solve the Problems

In order to accomplish the object as described above, the present invention is related to a motion capture system which detects body motions of a wearer comprising: a plurality of body motion sensors which are worn on arbitrary parts of the body of the wearer so that three-dimensional displacement and acceleration of each of the parts can be measured; a capturing unit configured to capture and record the body motion of the wearer; an output device configured to display detection results by the body motion sensors as body motion data in synchronization with a video image recorded by the capturing unit in order to make the body motion data and the video image comparable; and a synchronization processing unit configured to performs a synchronization process to make the time axis for displaying the body motion data and the time axis for displaying the motion image coincide with each other, wherein the synchronization processing unit performs the synchronization process by extracting a predetermined distinctive action by the wearer to invoke reaction of the body motion sensor from the motion image or sound data, extracting distinctive reaction corresponding to the distinctive action from the body motion data, and synchronizing the timing of the distinctive action and the timing of the distinctive reaction with each other.

The present invention is related to a motion capture system which detects body motions of a wearer comprising: a plurality of body motion sensors which are worn on arbitrary parts of the body of the wearer so that three-dimensional displacement and acceleration of each of the parts can be measured; a capturing unit configured to capture and record the body motion of the wearer; an output device configured to display detection results by the body motion sensors as body motion data in synchronization with a video image recorded by the capturing unit in order to make the body motion data and the video image comparable; a calibration signal transmission unit configured to transmit a calibration signal by outputting light emission or a sound signal; a calibration control signal acquisition unit configured to acquire a calibration control signal indicating the timing when the calibration signal is transmitted; and a synchronization processing unit configured to performs a synchronization process to make the time axis for displaying the body motion data and the time axis for displaying the motion image coincide with each other, wherein the synchronization processing unit performs the synchronization process by extracting the calibration signal transmitted by the calibration signal transmission unit from the video image or sound, and coinciding the timing of the calibration signal extracted from the video image or the sound signal with the timing indicated by the calibration control signal acquired by the calibration control signal acquisition unit.

The present invention is related to a motion capture method which detects body motions of a wearer comprising:
- a body motion recording step of measuring three-dimensional displacement and acceleration of each of arbitrary parts of the body of the wearer by body motion sensors which are worn on the arbitrary parts respectively, and recording detection results of the body motion sensors in a body motion recording unit as body motion data;
- a capturing step of capturing and recording the body motion of the wearer by a capturing unit; and
- an outputting step of performing a synchronization process to make the time axis for displaying the detection results by the body motion sensors as body motion data and the time axis for displaying a motion image recorded by the capturing unit coincide with each other, and displaying the body motion data synchronized to make the body motion data and the video image comparable,
   wherein, in the outputting step, the synchronization processing unit performs the synchronization process by extracting a predetermined distinctive action by the wearer to invoke reaction of the body motion sensor from the motion image or sound data, extracting distinctive reaction corresponding to the distinctive action from the body motion data, and synchronizing the timing of the distinctive action and the timing of the distinctive reaction with each other.

Also, the present invention is related to a motion capture method which detects body motions of a wearer comprising:
- a body motion recording step of measuring three-dimensional displacement and acceleration of each of arbitrary parts of the body of the wearer by body motion sensors which are worn on the arbitrary parts respectively, and recording detection results of the body motion sensors in a body motion recording unit as body motion data;
- a capturing step of capturing and recording the body motion of the wearer by a capturing unit; and
- a calibration control signal acquisition step of acquiring, by a calibration control signal acquisition unit, a calibration control signal indicating the timing when the calibration signal is transmitted by a calibration signal transmission unit which transmits the calibration signal by outputting light emission or a sound signal; and
- an outputting step of performing a synchronization process to make the time axis for displaying the detection results by the body motion sensors as body motion data and the time axis for displaying a motion image recorded by the capturing unit coincide with each other, and displaying the body motion data synchronized to make the body motion data and the video image comparable,
   wherein, in the outputting step, the synchronization processing unit performs the synchronization process by extracting the calibration signal transmitted by the calibration signal transmission unit from the video image or the sound signal, and coinciding the timing of the calibration signal extracted from the video image or sound with the timing indicated by the calibration control signal acquired by the calibration control signal acquisition unit.

Incidentally, the system and the method in accordance with the present invention as described above can be implemented in a computer by running the program of the present invention described in a predetermined language. Namely, the program in accordance with the present invention is a pace setting program which presents information about speed to a moving body by the use of a plurality of information processing terminals having a communication function and an information output function, and makes the information processing terminal function as a cooperation control unit which controls the timing to output the information about speed by the information output function of each information processing terminal through the communication function of each of the plurality of information processing terminals.

The system having the functionality as described above can be built to implement the method of the present invention by installing the program of the present invention in an IC chip or a memory device of a mobile terminal device, a smartphone, a wearable terminal, a tablet PC, another type information processing terminal, or a general purpose computer such as a personal computer or a server computer, and running the program on the CPU.

Namely, the present invention is related to a motion capture program for detecting body motions of a wearer, causing an information processing terminal to function as: a body motion recording unit configured to record detection results of a plurality of body motion sensors which are worn on arbitrary parts of the body of the wearer so that three-dimensional displacement and acceleration of each of the parts can be measured; an output device configured to display detection results by the body motion sensors as body motion data in synchronization with a video image recorded by a capturing unit which captures and records the body motion of the wearer in order to make the body motion data and the video image comparable; and a synchronization processing unit configured to performs a synchronization process to make the time axis for displaying the body motion data and the time axis for displaying the motion image coincide with each other, wherein the synchronization processing unit performs the synchronization process by extracting a predetermined distinctive action by the wearer to invoke reaction of the body motion sensor from the motion image or sound data, extracting distinctive reaction corresponding to the distinctive action from the body motion data, and synchronizing the timing of the distinctive action and the timing of the distinctive reaction with each other.

The present invention is related to a motion capture program for detecting body motions of a wearer, causing an information processing terminal to function as: a plurality of body motion sensors which are worn on arbitrary parts of the body of the wearer so that three-dimensional displacement and acceleration of each of the parts can be measured; a capturing unit configured to capture and record the body motion of the wearer; an output device configured to display detection results by the body motion sensors as body motion data in synchronization with a video image recorded by the capturing unit in order to make the body motion data and the video image comparable; and a calibration control signal acquisition unit configured to acquire a calibration control signal indicating the timing when the calibration signal is transmitted by a calibration signal transmission unit which transmits the calibration signal by outputting light emission or a sound signal; and a synchronization processing unit configured to performs a synchronization process to make the time axis for displaying the body motion data and the time axis for displaying the motion image coincide with each other, wherein the synchronization processing unit performs the synchronization process by extracting the calibration signal transmitted by the calibration signal transmission unit from the video image or the sound signal, and coinciding the timing of the calibration signal extracted from the video image or the sound signal with the timing indicated by the calibration control signal acquired by the calibration control signal acquisition unit.

### EFFECT OF THE INVENTION

As has been discussed above, in accordance with the present invention, the body motion of the wearer is recorded after adding a predetermined distinctive action by the wearer for invoking reaction of the body motion sensor to the video image, or recording video or audio transmission of a calibration signal by light emission or a sound signal. Then, the process of synchronizing the time axis for displaying the body motion data and the time axis for displaying the motion image with each other is performed by extracting the recorded distinctive action or calibration signal from the video image or sound, and making the timing of the distinctive action or calibration signal and the timing of the calibration control signal coincide with each other. By this configuration, it is possible to display the body motion data as recorded and the video image as captured in synchronism with each other in a comparable manner. As a result, in accordance with the present invention, even in the case where a slight time lag may occur between the replayed motion image and the displayed motion parameters depending upon the characteristics of the photographing equipment and the motion image process, it is possible to display/output the body motion of athletic activity in accurate synchronization with the video image, and perform appropriate training and coating by improving the analysis accuracy of the body motion.

### BRIEF DESCRIPTION OF DRAWINGS

[Fig. 1] Fig. 1 is an explanatory view for showing the usage of a motion capture system in accordance with a first and second embodiments.
[Fig. 2] Fig. 2 shows one example of body motion reproduction data which is acquired in accordance with the first and second embodiments.
[Fig. 3] Fig. 3 is a block diagram for showing the internal structure of the respective devices in accordance with the first embodiment.
[Fig. 4] Fig. 4 is a sequence diagram for showing the motion capture method in accordance with the first embodiment.
[Fig. 5] Fig. 5 is a flow chart for showing the data playback process of the motion capture method in accordance with the first embodiment.
[Fig. 6] Fig. 6 is a screen configuration view for showing the display screen (separate waveforms) of the motion capture system in accordance with the first and second embodiments.
[Fig. 7] Fig. 7 is a screen configuration view for showing the display screen (overlapped waveforms) of the motion capture system in accordance with the first and second embodiments.
[Fig. 8] Fig. 8 is a block diagram for showing the internal structure of the respective devices in accordance with the second embodiment.
[Fig. 9] Fig. 9 is a sequence diagram for showing the motion capture method in accordance with the second embodiment.
[Fig. 10] Fig. 10 is a flow chart for showing the synchronization process of the motion capture method in accordance with the second embodiment.

### BEST MODE FOR CARRYING OUT THE INVENTION

### [First Embodiment]

In what follows, a first embodiment of the present invention will be explained with reference to the accompanying drawings. The present embodiment provides a system which makes it possible to apply the present invention to a bicycle race and perform the body motion measurement and coaching with respect to the bicycle race by the use of an information terminal device 100. Incidentally, the embodiment described below has been disclosed with devices and the like only by way of illustration for implementing the technical idea of the present invention, which is not limited to the material, formation, structure, arrangement of each constituent member as described below. The technical idea of the present invention can be modified within the scope of claims.

### (System Configuration of Motion Capture System)

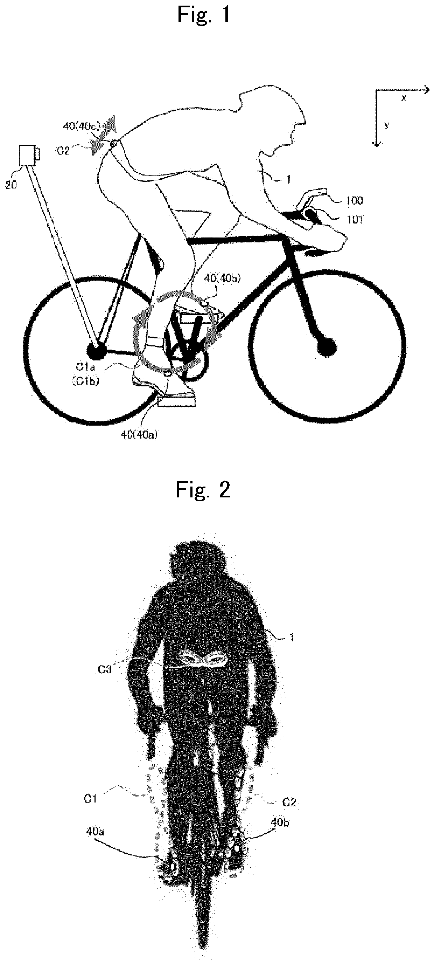

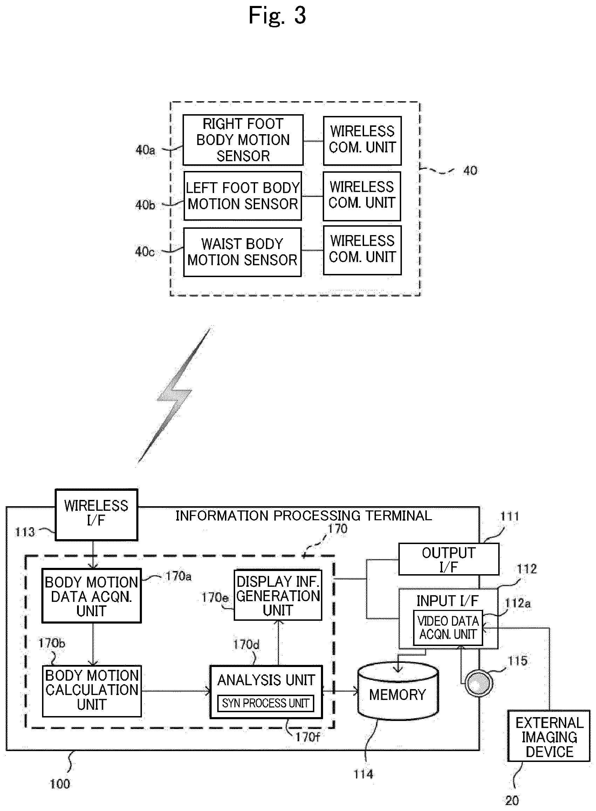

Fig. 1 is an explanatory view for showing the usage of a motion capture system with the information terminal device 100 in accordance with the present embodiment. Fig. 2 shows one example of body motion reproduction data which is acquired by the motion capture system in accordance with the present embodiment. Also, Fig. 3 is a block diagram for showing the internal structure of the respective devices.

As illustrated in Fig. 1 through Fig. 3, the motion capture system in accordance with the present embodiment includes the information terminal device 100 worn on a wearer 1, and a body motion sensors 40 (40a through 40c) worn on parts of the wearer and connected to the information terminal device 100 through wireless connection. Meanwhile, in the case of the present embodiment, a system can basically be implemented within an area where short range wireless communication is available between the information terminal device 100 and the body motion sensors 40. The system is not connected to a server or the like on the communication network during actual measurement, and can be operated offline in a so-called stand-alone configuration.

### (Structure of Each Device)

### (1) Body Motion Sensors

The body motion sensors 40a through 40c are sensors which are worn on parts of the wearer 1 taking exercise to detect the three-dimensional displacement and acceleration of each part. In the case of the present embodiment, the body motion sensors 40 include a right foot body motion sensor 40a worn on the right instep of the wearer, a left foot body motion sensor 40b worn on the left instep of the wearer, and a waist body motion sensor 40c worn on the waist of the wearer. Each of the motion sensors 40a through 40c incorporates a three-axis acceleration meter for measuring the acceleration of an object, a three-axis gyroscope for measuring the angular speed of the object, a three-axis magnetic sensor for measuring the magnitude and direction of a magnetic field, so that motions about nine axes can be detected. Each motion sensor 40 can be detachably worn on the shoes, belt, clothing of a wearer or the like with a clip or the like member, and the wearer can easily attach and detach each sensor for measurement, so that it is possible to perform continuous measurement without putting burdens on the wearer.

Each of these body motion sensors 40 (the body motion sensors 40a through 40c) is provided with a wireless communication unit as illustrated in Fig. 3. This wireless communication unit incorporates an antenna and can perform a communication process with the information terminal device 100 by the function to perform short range wireless communication on the basis of a data communication protocol such as BTLE (Bluetooth (registered trademark) Low Energy, Bluetooth (registered trademark) 4.0). Meanwhile, while the wireless communication unit of each body motion sensor 40 uses BTLE as a low power consumption communication protocol in the case of the present embodiment, ANT, ANT+ or the like protocol can be employed instead. Alternatively, standard Bluetooth (registered trademark) can be employed instead.

### (2) Information Terminal Device

Fig. 3 shows the internal structure of an information terminal device in accordance with the present embodiment. As illustrated in Fig. 1, the information terminal device 100 is a small-sized terminal device such as a smartphone which is used by being mounted on the handlebar of a bicycle or the like in order that another function can be used during traveling. However, in the case where the information terminal device 100 is used only to record body motion data during traveling, the information terminal device 100 can be put away in a storing implement such as a bag.

The information terminal device 100 is provided with a wireless interface 113, a control unit 170, a memory 114, an output interface 111 and an input interface 112 as illustrated in Fig. 3. More specifically, the information terminal device 100 in accordance with the present embodiment is provided with the function to collect detection results obtained by each body motion sensor 40 by obtaining the detection results through a communication process between each motion sensor 40 and the wireless communication interface 113. The memory 114 of the information terminal device 100 serves as a body motion recording unit which can record the detection results of the body motion sensors 40 as body motion data. In this description, the body motion data is raw data detected by each of various sensors, and the body motion reproduction data is data which is obtained by recording and analyzing this body motion data, extracting necessary data, performing correction and so forth.

Incidentally, the detection results as transmitted from each body motion sensor 40 include sensor identification information which is added for identifying this each body motion sensor 40 and accumulated in the memory 114 of the information terminal device 100 so that when acquiring a detection result through the wireless communication interface 113, the control unit 170 can determine which body motion sensor 40 outputs that detection result. Also, this identification information of each sensor includes wearing part identification information for identifying the part on which this each sensor is worn, and body motion reproduction data can be calculated on the basis of this wearing part identification information. Furthermore, the body motion data includes time information when the detection result is acquired from each body motion sensor 40.

The wireless communication interface 113 is a module for controlling transmission and reception of various information through the communication network and controlling wireless short-range communication such as wifi or Bluetooth (registered trademark) to communicate with each body motion sensor 40 by the use of one of various protocols and perform data transmission and reception with the above server device and the like through 3G communication.

Furthermore, the information terminal device 100 is provided with the output interface 111 and the input interface 112. The input interface 112 is a device such as a mouse, a keyboard, an operation button, a touch panel and the like for inputting user operation. On the other hand, the output interface 113 is a device such as a display, a speaker and the like for outputting images and sounds. Particularly, this output interface 113 includes a display unit such as a liquid crystal display on which a touch panel is placed as an input interface.

The display unit connected to the output interface 111 is an output device configured to display or output an analysis result of body motion reproduction data such that display information generated by a display information generation unit 170e is displayed through the output interface 111. The information is displayed in a display incorporated in the information terminal device 100 or an externally connected display.

On the other hand, the input interface 112 is provided with a video data acquisition unit 112a. The video data acquisition unit 112a is a module for acquiring video data which is captured and recorded as the body motion of the wearer. The video data acquired by the video data acquisition unit 112a includes video data in which motion images are recorded, audio data recorded together with the motion images, and meta data containing the capturing time, end time, time stamps for tracking the time elapsed and the like. The input interface 112 can be connected to a built-in camera 115 incorporated in the information terminal device 100 and an external camera 20 prepared separately. The video data captured by these capturing units is acquired by a video data acquisition unit 112a, accumulated in the memory 114 and processed by the control unit 170. Incidentally, the video data captured by the external camera 20 includes streaming data successively acquired on a real time base during capturing and video data downloaded after it is captured and stored by the external camera 20 as a file of the video data.

Furthermore, the information terminal device 100 of the present embodiment has the function to analyze the body motions of a wearer on the basis of body motion data acquired from the sensors, and generate the body motion reproduction data. Specifically, as illustrated in Fig. 3, the information terminal device 100 is provided with the control unit 170 which is an arithmetic processing unit such as a CPU for performing arithmetic operations required for controlling the respective elements. Incidentally, the respective functions of the information terminal device 100 are virtually implemented within the control unit 170 by running a motion capture program of the present invention with this control unit 170. More specifically, a body motion data acquisition unit 170a, a body motion calculation unit 170b, an analysis unit 170d and a display information generation unit 170e are virtually implemented by running a motion capture application with the control unit 170.

The body motion data acquisition unit 170a is a module for acquiring body motion data from each body motion sensor 40 through the wireless interface 113. In the case of the present embodiment, the body motion data acquisition unit 170a acquires the body motion data as the detection result by performing wireless communication with each of the body motion sensor 40a through 41c. This body motion data is temporarily accumulated in the memory 114, and then the detection results of the body motion sensors 40 are transmitted to the body motion calculation unit 170b.

The body motion calculation unit 170b is a module for calculating body motions of a wearer as body motion reproduction data on the basis of the detection results of the body motion sensors 40a through 40c accumulated in the memory 114 (body motion recording unit) and the displacement, rotation and acceleration of each of the body motion sensors 40a through 40c. In this case, the detection results of the body motion sensors 40 are values which are measured with a so-called nine-axis sensor and, in the case of the present embodiment, include the direction and magnitude of acceleration (including gravitational acceleration) of an object, an angular velocity (magnitude, direction and center position) of the object, and the direction (orientation) and magnitude of a magnetic field.

The body motions as calculated here include a factor indicative of the smoothness of pedaling, the angular velocity ω of the pedal shaft of a bicycle, the timely variation of this angular velocity ω, and the smoothness of the timely variation. Meanwhile, in the case of the present embodiment, the body motion sensors 40a and 40b are attached to right and left shoes so that the rotational motions that are detected by the sensors are the rotations of the shoes as illustrated in Fig. 2. However, when pedaling a bicycle, the shoes rotate on the pedal shaft so that ω is the angular velocity of this pedal shaft. On the other hand, the waist body motion sensor 40c is attached to the back side of the belt of the wearer, and laterally pivoted in synchronization with the motion of pedaling.

Furthermore, in the case of the present embodiment, the body motion calculation unit 170b extracts a cyclic change contained in body motion on the basis of the body motion reproduction data accumulated in the memory 114. The cyclic change includes not only a simple circular motion but also three-dimensional complicated free orbital locuses C1 through C3 in which are combined wavelike motions and motions in the shape of a figure eight as illustrated in Fig. 2. These orbital locuses are output as wave forms represented by amplitude and time. Specifically, in the case of the present embodiment, the extracted orbital motions include an orbital motion C1 of the right foot body motion sensor 40a worn on the right instep of the wearer 1, an orbital motion C2 of the left foot body motion sensor 40b worn on the left instep of the wearer 1, and a pivoting motion C3 of the waist of the wearer 1.

Meanwhile, in the case of the present embodiment, the body motion calculation unit 170b calculates the body motion of the wearer as body motion reproduction data on the basis of the detection results of the body motion sensors 40 and variation characteristics of orbital motions of the body motion sensors 40. In this case, for example, the body motion calculation unit 170b calculates three-dimensional free orbital locuses C1 through C3 of the body motion sensors 40 as illustrated in Fig. 2. Then, the body motion calculation unit 170b evaluates the amplitude, timing and collapse of the orbital and pivoting motions of the parts and the like in the left and right feet of the wearer on the basis of the calculated variation characteristics of the free orbital locuses C1 through C3. The body motion reproduction data is then calculated on the basis of the free orbital locuses C1 through C3 calculated in this manner.

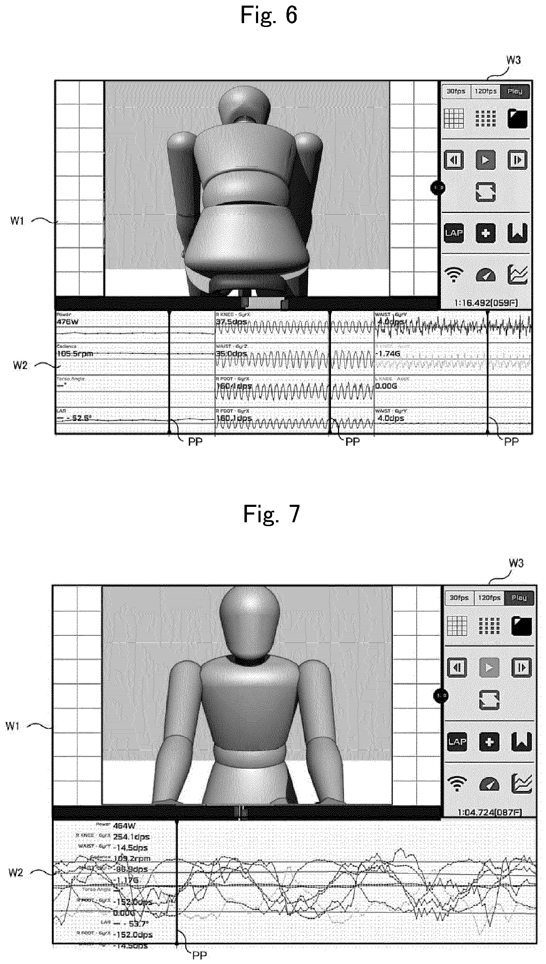

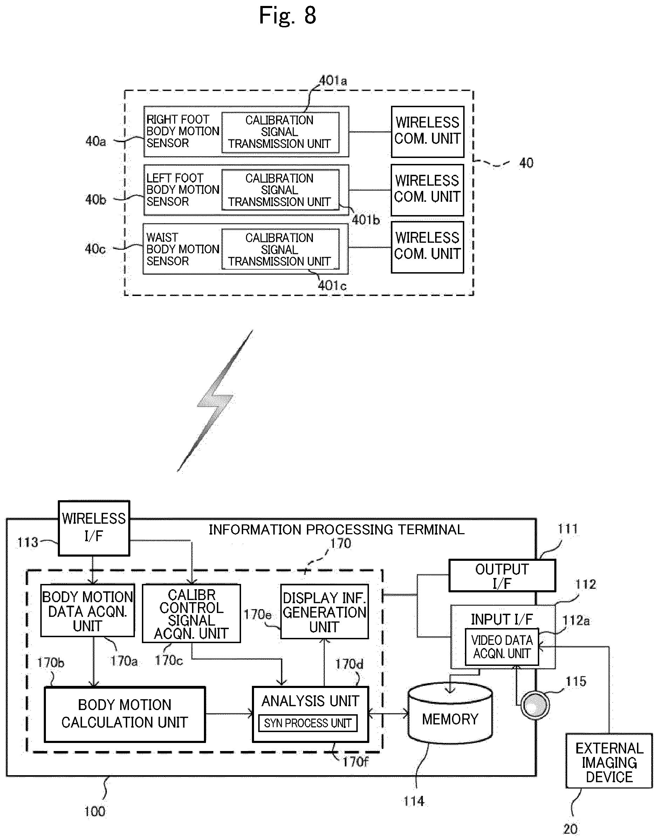

The analysis unit 170d is a module for analyzing each element of the body motions of the wearer 1 for each data item on the basis of body motion reproduction data. In the case of the present embodiment, the analysis unit 170d functions as a characteristic analysis unit which analyzes the characteristics of the angular velocity variation of cyclic motion, the amplitude and fluctuation of pivoting motion extracted by the body motion calculation unit 170b. The characteristics analyzed here are represented in waveforms on a timeline W2 defined with amplitude-time axes as illustrated in Fig. 7 and Fig. 8, and displayed or output by an output device through the display information generation unit 170e in synchronization with the video data.

Specifically, the analysis unit 170d analyzes the motion of peddling a bicycle, so-called pedaling. In particular, on the basis of the relative displacement and the variation of the angular velocity/acceleration of each sensor, the pedal crank, the bony framework of the wearer and the like, the body motion reproduction data is analyzed to estimate the time variation of force (power), the rhythm (Cadence), the angular variation of the upper half of the body (torso angle), the angular variation of the movable region of the thigh (LAR : Leg Angular Range), the displacements of the feet and knees in X direction and Y direction and the displacement of the waist in X direction and Y direction.

Meanwhile, as an alternative analyzing method, this analysis unit 170d may generate stereoscopic image data for three-dimensionally displaying the wearer 1, or generate two-dimensional image data by projecting the wearer 1 on a X-Y plane. Also, for example, improvement data indicative of the difference from a normal body motion is generated for example by extracting model body motion data from the memory 114 storing such model body motion data and comparing the body motion reproduction data of the wearer with the model body motion data. Furthermore, by registering user information in advance such as gender, height, weight and age, analysis may be performed on the basis of the user information. Then, the analysis unit 170d transmits the analysis results such as this stereoscopic image data and improvement data to the information terminal device 100.

Furthermore, in the case of the present embodiment, the analysis unit 170d is provided with a synchronization processing unit 170f. The synchronization processing unit 170f is a module for performing a calibration process to perform a synchronization process so that the time axis for displaying body motion data and the time axis for displaying the motion image captured by a camera coincide with each other. Specifically, the synchronization processing unit 170f according to the present embodiment performs a synchronization process by extracting a predetermined distinctive action (calibration action) by the wearer 1 to invoke reaction of the body motion sensor 40 from a motion image or sound data, extracting distinctive reaction corresponding to the distinctive action from the body motion data, and synchronizing the timing of the distinctive action and the timing of the distinctive reaction. This distinctive action includes applying vibrations predetermined times to the body motion sensor 40 within a short period, for example, by hitting or shaking the own body of the wearer 1 or the body motion sensor 40 itself predetermined times in front of the camera.

Such a process to extract a distinctive action is performed, for example, by detecting displacement of a figure having a predetermined profile or color with a certain amplitude or rhythm in a predetermined time period through an image recognition process to recognize an action of "hitting with a hand" by the wearer 1, reading the imaging time of the frame of the motion image corresponding to the maximum displacement during the action, and detecting the time information as a calibration signal. Alternatively, in the case where the video data contains audio data, a calibration signal may be detected as time information when the hitting sound of the wearer 1 is extracted from the audio data.

Furthermore, in the case where the video data has a large size corresponding to long time recording, user operation may auxiliarily be used in order that the distinctive action is extracted only from a time period designated by the user operation. On the other hand, the distinctive reaction of the body motion sensor 40 as described above can be detected by scanning the detection value of each sensor implemented in the body motion sensor 40 such as the acceleration sensor and extracting a part where a reaction having no smaller than a certain amplitude is repeated predetermined times within a predetermined period. Incidentally, the distinctive action and the distinctive reaction can be detected by detecting one and then detecting the other in a scanning region which is narrowed with reference to the time stamp of the one detected in advance.

Then, in order to have the timing of the distinctive action and the timing of the distinctive reaction coincide with each other, a synchronization process is performed by matching the timestamp (timely information) when the distinctive action is done and the timestamp when the distinctive reaction is detected to align the playback starting times thereof with each other. At this time, in the case where there is a difference between the reaction and the action repeatedly performed, synchronization is established by extending the playback time of the video or the timeline of the body motion sensor to align the playback starting time and end time of the video data with those of the body motion reproduction data.

The display information generation unit 170e is a module for generating display information to be displayed through the output interface 111, and displays or outputs the display information as the body motion reproduction data analyzed by the analysis unit 170d in correspondence with the video data. In the case of the present embodiment, as illustrated in Fig. 6 and Fig. 7, this display information includes the video image captured by the built-in camera 115 and the external camera 20 and displayed in a window W1, and the timeline W2 for displaying the body motion reproduction data analyzed by the analysis unit 170d in a planar circle. The video image and the timeline W2 are displayed in synchronization with each other to make them comparable. Meanwhile, this display information also includes sound signals and other output control signals together with the display data.

Also, the display information further includes GUI (Graphical User Interface) W3 on which the touch panel can be operated as input operation to the input interface 112 in order to switch the display by the display information generation unit 170e. In the case of the example shown in the figure, as illustrated in Fig. 6, the video image of the wearer 1 captured from behind with the external camera 20 is displayed in the window W1, and the respective motion parameters contained in the body motion reproduction data are displayed individually in the timeline W2. Also, by switching the display mode, as illustrated in Fig. 7, the video image of the wearer 1 captured from front with the built-in camera 115 of the information terminal device 100 is displayed in the window W1, and the respective motion parameters contained in the body motion reproduction data are displayed overlappingly in the timeline W2. Meanwhile, there are a variety of display modes which can be switched over, for example, a display mode in which the video image is displayed in a full screen view on which the timeline is overlapped.

In addition, when displaying the video image and the body motion reproduction data synchronized with each other so as to make them comparable, as illustrated in Fig. 6 and Fig. 7, a playback point PP is displayed on the timeline and can be slid in the right and left direction in order to display the frame of the video image in the time point indicated by the playback point PP on the timeline. The playback point PP moves on the timeline in synchronization with the playback of the video image to indicate the time progress, and can be used to designate a playback start point from which the video image is played back. Also, by setting a predetermined time slot, it is possible to play back the video image repeatedly in the time slot.

The memory 114 is a storage unit for storing various data such as the identification information for identifying each information terminal device 100, the wearing part identification information of each body motion sensor 40, the relative positional relationship of the body motion sensors 40 worn on the respective positions, the user information, the exemplar body motion data and the like.

### (Motion Capture Method)

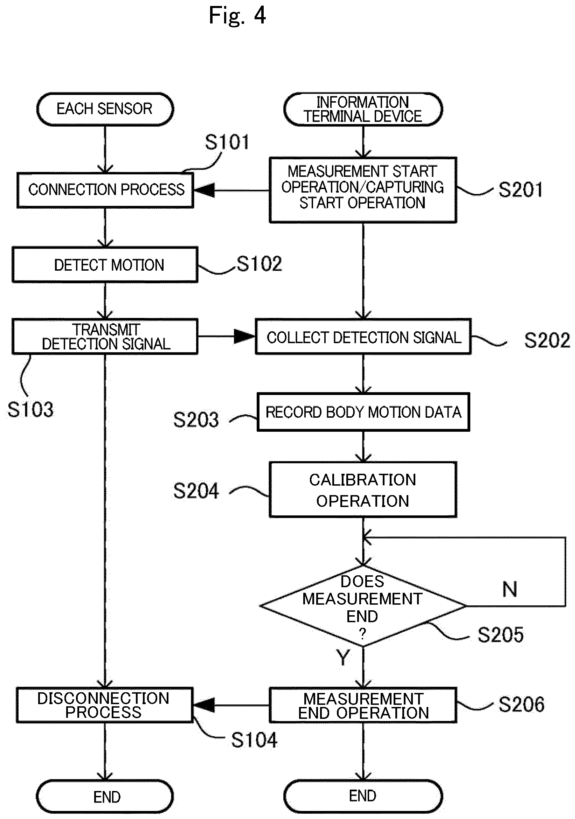

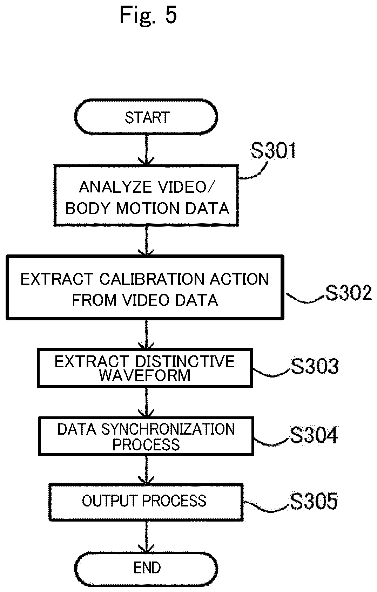

The motion capture method in accordance with the present invention can be implemented by operating the motion capture system having the structure as described above. Fig. 4 shows a recording operation, and Fig. 5 shows the operation during playing back the data.

### (1) Recording Operation

First, the wearer 1 wears the body motion sensors 40a through 40c on arbitrarily parts such as both feet and waist. Also, the wearer 1 performs motion capturing by fixing the information terminal device 100 to the handlebar 102 of a bicycle through a mount device and mounting the external camera 20 on the rear part of the bicycle.

Then, the application is invoked on the information terminal device 100 side as a program of the present invention, followed by inputting a measurement start operation to the application for acquiring a detection result of each body motion sensor 40, and performing a capturing start operation of the external camera 20 (S201). The control unit 170 of the information terminal device 100 performs a connection process with each body motion sensor 40 in response to the measurement start operation (S101). After performing the connection process, each motion sensor 40 starts detecting the motion of the wearer 1 (SI02). Specifically, the three-dimensional displacement or acceleration at each part of the wearer is detected by the body motion sensor 40 worn on this each part.

Then, the detection results which are acquired are transmitted to the wireless interface 113 of the information terminal device 100 from the wireless communication unit of each body motion sensor 40 by using weak electric waves (S103). When the wireless interface 113 of the information terminal device 100 starts acquiring the detection results (S202), the memory 114 serving as a body motion recording unit successively stores the detection results of the body motion sensors 40a through 40c as body motion data (S203).

Then, in advance of starting a competition such as a bicycle race, the operator performs a calibration operation (S204). Specifically, for example, the wearer 1 performs an action such as applying vibrations predetermined times to the body motion sensor 40 within a short period by hitting or shaking the own body of the wearer 1 or the body motion sensor 40 itself the predetermined times in front of the camera. Next, a competition is started, and the process of acquiring the detection values of the body motion sensor 40 and the process of recording the video image are continuously performed during the competition as well as the process storing the data in the memory 114 or the like ("N" in S206).

Specifically, the video data acquisition unit 112a acquires video data captured by the built-in camera 115 installed in the information terminal device 100 and the external camera 20 connected to the outside, and the video data is accumulated in the memory 114 and processed by the control unit 170. At this time, the detection data by the body motion sensor 40 and the video image as captured may be analyzed on a real time base and displayed by the display unit of the information terminal device 100. Thereafter, when the competition ends, the measurement is terminated ("Y" in S205), and if necessary, the recording process is halted (S206). Then, the communication with each sensor is disconnected (S104).

### (2) Playback Synchronization Process

The video data and the body motion data recorded as described above are played back as follows. Namely, as illustrated in Fig. 5, after the body motion data acquisition unit 170a acquires body motion data and the video data acquisition unit 112a acquires video data, the body motion calculation unit 170b and the analysis unit 170d calculates and analyzes the body motion of the wearer as body motion reproduction data on the basis of the detection results of the body motion sensors 40 accumulated on the memory 114 and the relative positional relationship among the body motion sensors 40 (S301).

Next is the process to extract a calibration action by the wearer 1 from the playback video and distinctive waveforms from the body motion data (S302 and S303). Specifically, the synchronization processing unit 170f extracts a predetermined distinctive action by the wearer 1 to make the body motion sensor 40 react from the video image or sound, and extracts a distinctive reaction from the body motion data. For example, the distinctive action is extracted by detecting displacement of a figure having a predetermined profile or color with a certain amplitude or rhythm in a predetermined time period through an image recognition process to recognize an action of "hitting with a hand" by the wearer 1, reading the imaging time of the frame of the motion image corresponding to the maximum displacement during the action, and detecting the time information as a calibration signal. Also, in the case where the video data contains sound data, a calibration signal may be detected as time information when the hitting sound of the wearer 1 is extracted from the audio data. On the other hand, the distinctive reaction of the body motion sensor 40 as described above can be detected by scanning the detection value of each sensor implemented in the body motion sensor 40 such as the acceleration sensor and extracting a part where a reaction having no smaller than a certain amplitude is repeated predetermined times within a predetermined period.

Next, the synchronization process of the video image and the body motion data is performed by synchronizing the timing of the distinctive action and the timing of the distinctive reaction (S305). Specifically, the synchronization process is performed by matching the timestamp (timely information) when the distinctive action is done and the timestamp when the distinctive reaction is detected to align the playback starting times thereof with each other. At this time, in the case where there is a difference between the reaction and the action repeatedly performed, synchronization is established by extending the playback time of the video or the timeline of the body motion sensor to align the playback starting time and end time of the video data with those of the body motion reproduction data. As has been discussed above, the video image and the body motion reproduction data analyzed and synchronized to make them comparable are displayed or output by the display or speaker of the information terminal device 100 as analysis results with sound and the like (S305).

### (Motion Capture Program)

Incidentally, the motion capture system and the motion capture method in accordance with the present embodiment as described above can be implemented in a computer by running the motion capture program of the present invention described in a predetermined language is the same as the above described motion capture application. Namely, the system having the functionality as described above can be built to implement the motion capture method by installing the program of the present invention in an IC chip or a memory device of a mobile terminal device, a smartphone, a wearable terminal, a mobile PC, another type information processing terminal, or a general purpose computer such as a personal computer or a server computer, and running the program on the CPU.

### (Effect/Action)

In accordance with the present embodiment as discussed above, the process of synchronizing the time axis for displaying the body motion data and the time axis for displaying the motion image with each other is performed by adding a calibration action (a predetermined distinctive action) for invoking reaction of the body motion sensor 40 to the video image in advance of recording the body motion of the wearer 1, extracting the recorded distinctive action from the video image or sound, and making the timing of the distinctive action and the timing of a calibration control signal coincide with each other. By this configuration, in accordance with the present embodiment, it is possible to display the body motion data as recorded and the video image as captured in synchronism with each other in a comparable manner, display/output the body motion of athletic activity in accurate synchronization with the video image, and perform appropriate training and coating while improving the analysis accuracy of the body motion.

Also, the motion capture program in accordance with the present embodiment can be distributed, for example, through a communication line, or as a package application which can be run on a stand-alone computer by storing the program in a storage medium which can be read by a computer. Specifically, such a storage medium includes a magnetic recording medium such as a flexible disk or a cassette tape, an optical disc such as CD-ROM or DVD-ROM, a RAM card and a variety of storage mediums. In addition, in accordance with the computer readable medium in which this program is stored, the above system and method can be easily implemented with a general purpose computer or a dedicated computer, and the program can be easily maintained, transported and installed.

### [Second Embodiment]

Next, the second embodiment of the present invention will be explained. While the synchronization process between the captured video image and the body motion data is performed on the basis of the calibration action performed by the wearer 1 in the case of the first embodiment of the present invention as described above, the gist of the present embodiment resides in a calibration signal transmission unit installed in the body motion sensor 40 to automatically perform the synchronization process. Meanwhile, in the description of the present embodiment, like reference numbers indicate functionally similar elements as the above first embodiment unless otherwise specified, and therefore no redundant description is repeated.

### (Structure of Each Device)

### (1) Body Motion Sensors

In addition to the configuration explained in the first embodiment as described above, the body motion sensors 40a to 40c in accordance with the present embodiment are provided with calibration signal transmission units 401 (401a to 401c) respectively for transmitting calibration signals by outputting light emission or sound signals as illustrated in Fig. 8. This calibration signal transmission unit 401 outputs a calibration signal in the form of light or sound, for example, by providing a light emitting device such as an LED on the external surface of the sensor body in the visible state, or providing a sound generating means such as a speaker. The transmission of a calibration signal by the calibration signal transmission unit 401 is performed on the basis of a calibration execution signal transmitted from a calibration control signal acquisition unit 170c, performed on the basis of an arbitrary operation by the wearer 1, and so forth. The arbitrary operation by the wearer 1 is, for example, to push an operation button provided on the body motion sensor 40, transmit a control signal from another device and so forth. The calibration signal transmission unit 401 transmits, to the calibration control signal acquisition unit 170c, the time when a calibration signal is actually transmitted as a calibration control signal.

### (2) Information Terminal Device

In addition to the configuration explained in the first embodiment as described above, the information terminal device 100 in accordance with the present embodiment is provided with the calibration control signal acquisition unit 170c as illustrated in Fig. 8. This calibration control signal acquisition unit 170c transmits a calibration execution signal to the calibration signal transmission unit 401 to transmit a calibration signal from the calibration signal transmission unit 401, and acquires the time when the calibration signal is actually transmitted as a calibration control signal. This calibration control signal acquired by the calibration control signal acquisition unit 170c is input to the analysis unit 170d.

In the case of the present embodiment, the synchronization processing unit 170f extracts the calibration signal transmitted by the calibration signal transmission unit 401 from the video image or sound. In addition to this, the synchronization processing unit 170f performs a synchronization process by coinciding the transmission timing of the calibration signal extracted from the video image or sound with the actual transmission timing indicated by the calibration control signal acquired by the calibration control signal acquisition unit 170c.

### (Motion Capture Method)

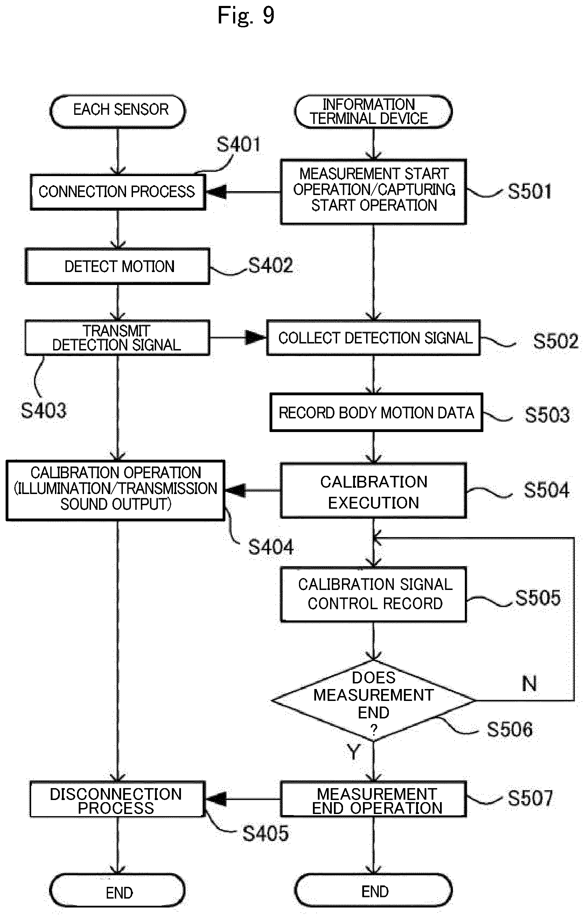

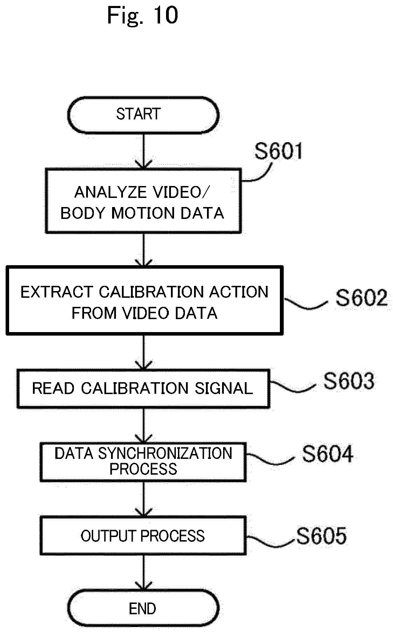

The motion capture method in accordance with the present invention can be implemented by operating the motion capture system having the structure as described above. Fig. 9 and Fig. 10 show the recording operation and the operation during playing back the data respectively in accordance with the second embodiment.

### (1) Recording Operation

First, the wearer 1 wears the body motion sensors 40a through 40c on arbitrarily parts such as both feet and waist. Also, the wearer 1 performs motion capturing by fixing the information terminal device 100 to the handlebar 102 of a bicycle through a mount device and mounting the external camera 20 on the rear part of the bicycle.

Then, the application is invoked on the information terminal device 100 side as a program of the present invention, followed by inputting a measurement start operation to the application for acquiring a detection result of each body motion sensor 40, and performing a capturing start operation of the external camera 20 (S501). The control unit 170 of the information terminal device 100 performs a connection process with each body motion sensor 40 in response to the measurement start operation (S401). After performing the connection process, each motion sensor 40 starts detecting the motion of the wearer 1 (S402). Specifically, the three-dimensional displacement or acceleration at each part of the wearer is detected by the body motion sensor 40 worn on this each part.

Then, the detection results which are acquired are transmitted to the wireless interface 113 of the information terminal device 100 from the wireless communication unit of each body motion sensor 40 by using weak electric waves (S403). When the wireless interface 113 of the information terminal device 100 starts acquiring the detection results (S502), the memory 114 serving as a body motion recording unit successively stores the detection results of the body motion sensors 40a through 40c as body motion data (S503).

Then, in advance of starting a competition such as a bicycle race, the calibration operation is performed (S504). In the case of the present embodiment, the calibration operation is automatically performed by the calibration control signal acquisition unit 170c as the center on the information terminal device 100 side. Specifically, for example, the calibration signal transmission unit 401 outputs a calibration signal of light or sound on the basis of the calibration execution signal transmitted from the calibration control signal acquisition unit 170c. Alternatively, the transmission of the calibration signal from the calibration signal transmission unit 401 may be performed on the basis of an arbitrary operation by the wearer 1, or performed on the basis of a control signal output by another device. The calibration signal transmission unit 401 transmits, to the calibration control signal acquisition unit 170c, the time when a calibration signal is actually transmitted, which is received as the calibration control signal by the calibration control signal acquisition unit 170c and recorded in the memory 114 through the analysis unit 170d.

Next, a competition is started, and the process of acquiring the detection values of the body motion sensor 40 and the process of recording the video image are continuously performed during the competition as well as the process storing the data in the memory 114 or the like ("N" in S506). Specifically, the video data acquisition unit 112a acquires video data captured by the built-in camera 115 installed in the information terminal device 100 and the external camera 20 connected to the outside, and the video data is accumulated in the memory 114 and processed by the control unit 170. At this time, the detection data by the body motion sensor 40 and the video image as captured may be analyzed on a real time base and displayed by the display unit of the information terminal device 100. Thereafter, when the competition ends, the measurement is terminated ("Y" in S506), and if necessary, the recording process is halted (S507). Then, the communication with each sensor is disconnected (S405).

### (2) Playback Synchronization Process

The video data and the body motion data recorded as described above are played back as follows. Namely, as illustrated in Fig. 10, after the body motion data acquisition unit 170a acquires body motion data and the video data acquisition unit 112a acquires video data, the body motion calculation unit 170b and the analysis unit 170d calculates and analyzes the body motion of the wearer as body motion reproduction data on the basis of the detection results of the body motion sensors 40 accumulated on the memory 114 and the relative positional relationship among the body motion sensors 40 (S601).

Next, a calibration signal is extracted by detecting illumination of the body motion sensor 40 captured in the video image or transmission sound output (S602), and the transmission record of the calibration signal indicated by the calibration control signal is read from the memory 114 (S603). Specifically, the synchronization processing unit 170f extracts illumination or transmission sound output (calibration signal) from the video image or sound, and reads the transmission time of the calibration signal described in the calibration control signal. For example, the synchronization processing unit 170f recognizes emission of light having a predetermined wavelength with a certain amplitude or rhythm in a predetermined time period to detect the timely information as a calibration signal. Alternatively, in the case where the video data contains audio data, a calibration signal may be detected as time information when the sound signal transmitted from the calibration signal transmission unit 401 is extracted from the audio data.

Next, the synchronization process of the video image and the body motion data is performed by synchronizing the timing of the calibration signal as extracted and the timing of the calibration control signal (S605). Specifically, the synchronization process is performed by matching the timestamp (timely information) when the calibration signal is transmitted and the timestamp described in the transmission record of the calibration control signal to align the playback starting times thereof with each other. At this time, in the case where there is a difference between the reaction and the action repeatedly performed, synchronization is established by extending the playback time of the video or the timeline of the body motion sensor to align the playback starting time and end time of the video data with those of the body motion reproduction data. As has been discussed above, the video image and the body motion reproduction data analyzed and synchronized to make them comparable are displayed or output by the display or speaker of the information terminal device 100 as analysis results with sound and the like (S605).

### (Motion Capture Program)

Incidentally, the motion capture system and the motion capture method in accordance with the present embodiment as described above can be also implemented in a computer by running the motion capture program of the present invention described in a predetermined language is the same as the above described motion capture application. Namely, the system having the functionality as described above can be built to implement the motion capture method by installing the program of the present invention in an IC chip or a memory device of a mobile terminal device, a smartphone, a wearable terminal, a mobile PC, another type information processing terminal, or a general purpose computer such as a personal computer or a server computer, and running the program on the CPU.

### (Effect/Action)

In accordance with the present embodiment as discussed above, a calibration signal by light emission or a sound signal is automatically transmitted in advance of recording the body motion of the wearer 1, and the distinctive reaction in response to the calibration signal is video or audio recorded. Then, the process of synchronizing the time axis for displaying the body motion data and the time axis for displaying the motion image with each other is performed by extracting the recorded calibration signal from the video image or sound, and making the timing of the calibration signal and the timing of the calibration control signal coincide with each other. By this configuration, the body motion data as recorded and the video image as captured can be synchronized and displayed to make them comparable. As a result, in accordance with the present embodiment, even in the case where a slight time lag may occur between the replayed motion image and the displayed motion parameters depending upon the characteristics of the photographing equipment and the motion image process, it is possible to display/output the body motion of athletic activity in accurate synchronization with the video image, and perform appropriate training and coating by improving the analysis accuracy of the body motion.

Incidentally, the present invention is not limited to the above described embodiments as they are, but it is possible to modify the constituent elements for embodiment without departing from the sprit of the present invention. Also, a variety of inventions may be embodied by appropriately combining a plurality of constituent elements disclosed in the above described embodiments. For example, it is possible to dispense with some elements of all the constituent elements disclosed in the embodiments.

### EXPLANATION OF SYMBOLS

- C1 through C3: free orbital locus
- PP: playback point cursor
- W1: window
- W2: timeline
- W3: GUI
- X: travelling direction
- Y: vertical direction
- 1: wearer
- 20: external camera
- 40: body motion sensor
- 40a: right foot body motion sensor
- 40b: left foot body motion sensor
- 40c: waist body motion sensor
- 100: information terminal device
- 111: output interface
- 112: input interface
- 112a: video data acquisition unit
- 113: wireless interface
- 114: memory
- 115: built-in camera
- 170: control unit
- 170a: body motion data acquisition unit
- 170b: body motion calculation unit
- 170c: calibration control signal acquisition unit
- 170d: analysis unit
- 170e: display information generation unit
- 170f: synchronization processing unit
- 401: calibration signal transmission unit

## Claims

1. A motion capture system which detects body motions of a wearer comprising:
a plurality of body motion sensors which are worn on arbitrary parts of the body of the wearer so that three-dimensional displacement and acceleration of each of the parts can be measured;
a capturing unit configured to capture and record the body motion of the wearer;
an output device configured to display detection results by the body motion sensors as body motion data in synchronization with a video image recorded by the capturing unit in order to make the body motion data and the video image comparable; and
a synchronization processing unit configured to performs a synchronization process to make the time axis for displaying the body motion data and the time axis for displaying the motion image coincide with each other,
wherein the synchronization processing unit performs the synchronization process by extracting a predetermined distinctive action by the wearer to invoke reaction of the body motion sensor from the motion image or sound data, extracting distinctive reaction corresponding to the distinctive action from the body motion data, and synchronizing the timing of the distinctive action and the timing of the distinctive reaction with each other.

2. The motion capture system of claim 1 wherein
the distinctive action is to apply vibrations to the body motion sensor predetermined times within a short period.

3. A motion capture system which detects body motions of a wearer comprising:
a plurality of body motion sensors which are worn on arbitrary parts of the body of the wearer so that three-dimensional displacement and acceleration of each of the parts can be measured;
a capturing unit configured to capture and record the body motion of the wearer;
an output device configured to display detection results by the body motion sensors as body motion data in synchronization with a video image recorded by the capturing unit in order to make the body motion data and the video image comparable;
a calibration signal transmission unit configured to transmit a calibration signal by outputting light emission or a sound signal;
a calibration control signal acquisition unit configured to acquire a calibration control signal indicating the timing when the calibration signal is transmitted; and
a synchronization processing unit configured to performs a synchronization process to make the time axis for displaying the body motion data and the time axis for displaying the motion image coincide with each other,
wherein the synchronization processing unit performs the synchronization process by extracting the calibration signal transmitted by the calibration signal transmission unit from the video image or sound, and coinciding the timing of the calibration signal extracted from the video image or the sound signal with the timing indicated by the calibration control signal acquired by the calibration control signal acquisition unit.

4. A motion capture program for detecting body motions of a wearer, causing an information processing terminal to function as:
a body motion recording unit configured to record detection results of a plurality of body motion sensors which are worn on arbitrary parts of the body of the wearer so that three-dimensional displacement and acceleration of each of the parts can be measured;
an output device configured to display detection results by the body motion sensors as body motion data in synchronization with a video image recorded by a capturing unit which captures and records the body motion of the wearer in order to make the body motion data and the video image comparable; and
a synchronization processing unit configured to performs a synchronization process to make the time axis for displaying the body motion data and the time axis for displaying the motion image coincide with each other,
wherein the synchronization processing unit performs the synchronization process by extracting a predetermined distinctive action by the wearer to invoke reaction of the body motion sensor from the motion image or sound data, extracting distinctive reaction corresponding to the distinctive action from the body motion data, and synchronizing the timing of the distinctive action and the timing of the distinctive reaction with each other.

5. The motion capture program of claim 4 wherein
the distinctive action is to apply vibrations to the body motion sensor predetermined times within a short period.

6. A motion capture program for detecting body motions of a wearer, causing an information processing terminal to function as:
a plurality of body motion sensors which are worn on arbitrary parts of the body of the wearer so that three-dimensional displacement and acceleration of each of the parts can be measured;
a capturing unit configured to capture and record the body motion of the wearer;
an output device configured to display detection results by the body motion sensors as body motion data in synchronization with a video image recorded by the capturing unit in order to make the body motion data and the video image comparable; and
a calibration control signal acquisition unit configured to acquire a calibration control signal indicating the timing when the calibration signal is transmitted by a calibration signal transmission unit which transmits the calibration signal by outputting light emission or a sound signal; and
a synchronization processing unit configured to performs a synchronization process to make the time axis for displaying the body motion data and the time axis for displaying the motion image coincide with each other,
wherein the synchronization processing unit performs the synchronization process by extracting the calibration signal transmitted by the calibration signal transmission unit from the video image or the sound signal, and coinciding the timing of the calibration signal extracted from the video image or the sound signal with the timing indicated by the calibration control signal acquired by the calibration control signal acquisition unit.

7. A motion capture method which detects body motions of a wearer comprising:
a body motion recording step of measuring three-dimensional displacement and acceleration of each of arbitrary parts of the body of the wearer by body motion sensors which are worn on the arbitrary parts respectively, and recording detection results of the body motion sensors in a body motion recording unit as body motion data;
a capturing step of capturing and recording the body motion of the wearer by a capturing unit; and
an outputting step of performing a synchronization process to make the time axis for displaying the detection results by the body motion sensors as body motion data and the time axis for displaying a motion image recorded by the capturing unit coincide with each other, and displaying the body motion data synchronized to make the body motion data and the video image comparable,
wherein, in the outputting step, the synchronization process is performed by extracting a predetermined distinctive action by the wearer to invoke reaction of the body motion sensor from the motion image or sound data, extracting distinctive reaction corresponding to the distinctive action from the body motion data, and synchronizing the timing of the distinctive action and the timing of the distinctive reaction with each other.

8. The motion capture method of claim 7 wherein
the distinctive action is to apply vibrations to the body motion sensor predetermined times within a short period.

9. A motion capture method which detects body motions of a wearer comprising:
a body motion recording step of measuring three-dimensional displacement and acceleration of each of arbitrary parts of the body of the wearer by body motion sensors which are worn on the arbitrary parts respectively, and recording detection results of the body motion sensors in a body motion recording unit as body motion data;
a capturing step of capturing and recording the body motion of the wearer by a capturing unit; and
a calibration control signal acquisition step of acquiring, by a calibration control signal acquisition unit, a calibration control signal indicating the timing when the calibration signal is transmitted by a calibration signal transmission unit which transmits the calibration signal by outputting light emission or a sound signal; and
an outputting step of performing a synchronization process to make the time axis for displaying the detection results by the body motion sensors as body motion data and the time axis for displaying a motion image recorded by the capturing unit coincide with each other, and displaying the body motion data synchronized to make the body motion data and the video image comparable,
wherein, in the outputting step, the synchronization processing unit performs the synchronization process by extracting the calibration signal transmitted by the calibration signal transmission unit from the video image or the sound signal, and coinciding the timing of the calibration signal extracted from the video image or sound with the timing indicated by the calibration control signal acquired by the calibration control signal acquisition unit.
